# EUROPEAN PATENT SPECIFICATION

(11) **EP 4 180 055 B1**
(45) Date of publication and mention of the grant of the patent: **30.04.2025**
(21) Application number: 21854523.4
(22) Date of filing: 06.08.2021
(51) Int. Cl.: A61K 31/136, A61K 38/50, A61P 35/00, A61K 9/127

(54) **USE OF MITOXANTRONE HYDROCHLORIDE LIPOSOME AND PEGASPARGASE**
VERWENDUNG VON MITOXANTRONHYDROCHLORIDLIPOSOM UND PEGASPARGASE
UTILISATION DE LIPOSOME DE CHLORHYDRATE DE MITOXANTRONE ET PÉGASPARGASE

(30) Priority: 07.08.2020 CN 202010788006
(43) Date of publication of application: 17.05.2023
(73) Proprietor: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); YAN, Shujie, Shijiazhuang, Hebei 050035 (CN); LI, Yanhui, Shijiazhuang, Hebei 050035 (CN); LIN, Hongmei, Shijiazhuang, Hebei 050035 (CN); XIA, Xuefang, Shijiazhuang, Hebei 050035 (CN); ZHOU, Meng, Shijiazhuang, Hebei 050035 (CN); WANG, Shixia, Shijiazhuang, Hebei 050035 (CN); YANG, Shasha, Shijiazhuang, Hebei 050035 (CN)
(74) Representative: Gassner, Birgitta
(86) International application number: PCT/CN2021/111121
(87) International publication number: WO 2022/028566

(56) References cited:
- WO-A1-2020/029918
- CN-A- 110 711 178
- US-A1- 2009 306 103
- US-A1- 2018 208 597
- JIANPING LAI ET AL: "Successful treatment with anti-programmed-death-1 antibody in a relapsed natural killer/T-cell lymphoma patient with multi-line resistance: a case report", BMC CANCER, vol. 17, no. 1, 28 July 2017 (2017-07-28), pages 507, XP055590207, DOI: 10.1186/s12885-017-3501-4
- LIU WEIPING ET AL: "Treatment, Survival, and Prognosis of Advanced-Stage Natural Killer/T-Cell Lymphoma: An Analysis From the China Lymphoma Collaborative Group", FRONTIERS IN ONCOLOGY, vol. 10, 19 February 2021 (2021-02-19), XP093222290, ISSN: 2234-943X, DOI: 10.3389/fonc.2020.583050
- HU SHAOXUAN ET AL: "A Prospective Phase II Study of Pegaspargase-COEP Plus Radiotherapy in Patients With Newly Diagnosed Extra-Nodal NK/T-Cell Lymphoma", FRONTIERS IN ONCOLOGY, vol. 12, 25 February 2022 (2022-02-25), XP093222292, ISSN: 2234-943X, DOI: 10.3389/fonc.2022.839252
- WANG XINHUA ET AL: "Efficacy and Safety of a Pegasparaginase-Based Chemotherapy Regimen vs an L-asparaginase-Based Chemotherapy Regimen for Newly Diagnosed Advanced Extranodal Natural Killer/T-Cell Lymphoma : A Randomized Clinical Trial", JAMA ONCOLOGY, vol. 8, no. 7, 1 July 2022 (2022-07-01), US, pages 1035, XP093222296, ISSN: 2374-2437, DOI: 10.1001/jamaoncol.2022.1968
- AUGUST KEITH J., GUEST ERIN M., LEWING KAREN, HAYS J. ALLYSON, GAMIS ALAN S.: "Treatment of children with relapsed and refractory acute lymphoblastic leukemia with mitoxantrone, vincristine, pegaspargase, dexamethasone, and bortezomib", PEDIATRIC BLOOD AND CANCER, WILEY, HOBOKEN, NJ, US, vol. 67, no. 3, 1 March 2020 (2020-03-01), US , XP055894323, ISSN: 1545-5009, DOI: 10.1002/pbc.28062

## Description

### Technical field

The present invention belongs to the field of antitumor, specifically relates to mitoxantrone hydrochloride liposome and pegaspargase for the use in treating NK/T-cell lymphoma (NKTCL).

### Background art

NKTCL (NK/T-cell lymphoma) is clinically divided into nasal type (Extranodal NK/T-cell Lymphoma Nasal Type) and non-nasal type, and the nasal type is a rare type of aggressive non-Hodgkin lymphoma. The incidence of this disease is low, in proportion of about 11% to 14% of lymphoma in China (non-patent reference 1). Although there are geographical differences in the incidence of NKTCL, the characteristics of this disease are similar in endemic areas such as Western countries and East Asia. It mostly occurs in the upper respiratory tract and digestive tract, about 80% of which occurs in the nasal cavity, and can also occur in organs outside the nasal cavity such as Waldeyer's ring and nasopharynx, and occasionally in the skin, digestive tract, testicles, etc. Although about 67% to 80% of patients are early-stage (stage I/II), the disease is strongly invasive and has a poor prognosis, and the 5-year overall survival (Over Survival, OS) rate is about 32%; The median OS is 8 months, and the 5-year OS rate of early-stage-NKTCL is 25% to 86%. Tumor pathogenesis is closely related to Epstein-Barr virus (EBV) infection, and quantitative detection of plasma EBV-DNA can reflect the tumor burden, progression and prognosis. The large variation in patient's survival is mainly due to disease heterogeneity and different choices of treatment strategies. Most NKTCLs express mRNA of multi-drug resistance gene and its expression product P-glycoprotein (PgP), and produce multi-drug resistance (MDR), so that the CHOP or CHOP-like chemotherapy regimens based on anthracycline have the defects of low response rate, with complete response (CR) rate of 25% to 50%, 5-year OS rate of 0% to 34% (non-patent reference 2). Therefore, there is no literature reporting that mitoxantrone can be used for treating NKTCL, and the serious adverse reactions of mitoxantrone (bone marrow suppression, dose-limiting cardiotoxicity, gastrointestinal toxicity, and fatigue, etc.) limit its clinical use.

Liposome is a novel drug delivery system. Mitoxantrone liposomal preparations have been studied. For example, the Chinese patent application 200610102339.8 filed on December 29, 2006 and the PCT application WO2008/080367A1 filed on December 29, 2007, disclosed a mitoxantrone liposome, Studies have shown that the liposomal preparations have lower toxicity and better antitumor efficacy at lower doses compared with mitoxantrone free drug.

Antitumor drugs that are not affected by multi-drug resistance such as L-asparaginase (L-ASP), gemcitabine and the like are increasingly used in NKTCL, they have shown good efficacy whether in terms of CR rate, OS, or progression-free survival (PFS), and significant improvement of the prognosis of patients compared with CHOP or CHOP-like chemotherapy regimens. In particular, L-ASP can hydrolyze asparagine in serum, so that the syntheses of DNA, RNA and protein of tumor cells are inhibited, thereby playing a role of anti-tumor effect. Chemotherapy regimens containing L-ASP are effective in both patients in early diagnosed stage and patients in progressive, relapsed/refractory stages. The efficacy of L-ASP in NKTCL is significantly improved compared with anthracyclines, therefore, the chemotherapy containing L-ASP for NKTCL is significantly improved. For early NKTCL, the efficacy of the combination of radiotherapy + chemotherapy is higher than that of radiotherapy alone. The efficacy of the combination of the first course of radiotherapy followed by chemotherapy or the combination of the concurrent radiotherapy and chemotherapy is better than that of chemotherapy alone, with the 5-year OS rate 45% to 100%. A domestic study from two research centers reported that, comparing with the CHOP regimen for treating patients with early-stage (stage II.) NKTCL, the he efficacy of LOP regimen (L-ASP + vincristine + dexamethasone) was significantly better. The CR rates of the two groups were 68.8% and 50% respectively, the 3-year OS rates were 87.5% and 62.5% respectively, and the PFS rates were 79.2% and 50% respectively (non-patent reference 3). Since 2010, NCCN guidelines also recommend L-ASP-based combination chemotherapy as the first-line regimen for treating NKTCL. Although L-ASP-based chemotherapy regimens can significantly improve clinical efficacy and long-term survival, however, L-ASP is a heterologous protein derived from *Escherichia coli* that stimulates the body to produce antibodies, develop allergic reactions and life-threatening in severe cases, and reduces the efficacy of L-ASP and disease-free long-term survival.

In order to improve the efficacy and safety of L-ASP, *Escherichia coli*-derived L-ASP is linked to polyethylene glycol and lipid bilayer to form liposomal asparaginase PEG-ASP, so as to prolong the half-life, achieve low immunogenicity, significantly reduce allergic reactions, especially have less immediate allergic reactions, and other adverse reactions similar to L-ASP. Since 2013, PEG-ASP was recommended to replace L-ASP.

Domestic researchers applied a sandwich treatment regimen of pegaspargase, gemcitabine and oxaliplatin to newly diagnosed NKTCL patients, and it showed that a complete response (CR) rate was only 23.68%, partial response (PR) rate was 63.16%, and objective response rate (ORR) was 86.84% after at least 2 cycles (6 weeks) of treatment. After the completion of treatment, the ORR was 92.1% (CR rate was 86.84%, PR rate was 5.26%), 3-year OS rate and PFS rate were 72.6% and 57.8%, respectively, and grade 1/2 hematologic toxicity was common (non-patent references 4 and 5). Therefore, there is a need for improvements in combination chemotherapy regimens of pegaspargase.

However, due to the low incidence of NKTCL, there is a lack of large-scale randomized controlled studies on the treatment of the disease, and most of the current therapeutic evidence comes from retrospective analyses or early clinical trials (phase I/II) with small samples, which are still insufficient to form treatment recommendations with sufficient medical evidences. Therefore, it is difficult to improve the combination chemotherapy regimen of pegaspargase by referring to clinical data.

### List of cited documents

### Non-patent references

Non-patent reference 1: Li YX, et al. Radiotherapy alone with curative intent in patients with stage I extranodal nasal-type NK/T-cell lymphoma. Int J Radiat Oncol Biol Phys. 2012 Apr 1;82(5):1809-1815.

Non-patent reference 2: Isobe K, Uno T,Tamaru J, et al. Extranodal natural killer/T - cell lymphoma, nasal type: The significance of radiotherapeutic parameters. Cancer, 2006, 106: 609-615.

Non-patent reference 3: Hamaguchi M, et al.Phase II study of SMILE chemotherapy for newly diagnosed stage IV, relapsed, or refractory extranodal natural killer (NK)/T-cell lymphoma, nasal type: the NK-Cell Tumor Study Group study. J Clin Oncol. 2011 Nov 20; 29(33):4410-4416.

Non-patent reference 4: Jing XM, et al. Efficacy and tolerance of gemcitabine and oxaliplatin with sandwiched radio-therapy in the treatment of newly-diagnosed extra nodal nature (NK)/Tcell lymphoma. LeukRes. 2016, 47:26-31.

Non-patent reference 5: Wang JH, et al, Analysis of the efficacy and safety of a combined gemcitabine, oxaliplatin and pegaspargase regimen for NK/T-cell lymphoma. Oncotarget. 2016 Jun 7;7(23):35412-35422.

### Patent reference:

CN 110 711 178 A

### Summary of the invention

Unless otherwise specified, the terms of "treatment naïve", "relapsed", "refractory", "asparaginase/pegaspargase-containing chemotherapy regimen" in the present invention are defined as follows:
"Treatment naïve" is defined as being diagnosed for the first time, and has not been treated.
"Relapsed" is defined as the occurrence of new lesions at the primary site or other sites after the complete response (CR) is achieved with administrating asparaginase/pegaspargase-containing chemotherapy regimen.
"Refractory" is defined as any of the following situations: the efficacy evaluation is progressive disease (PD) after 2 cycles of treatment with applying asparaginase/pegaspargase-containing chemotherapy regimen, or the efficacy evaluation do not reach partial response (PR) after 4 cycles of treatment, or the efficacy evaluation do not reach complete response (CR) after 6 cycles of treatment.
"Asparaginase/pegaspargase-containing chemotherapy regimen" refers to the combination chemotherapy regimen of asparaginase or pegaspargase described in the background technique, such as SMILE regimen, GEMOX regimen, AspaMet-Dex regimen or LOP regimen.

The present invention provides mitoxantrone liposome and pegaspargase for use in treating NKTCL.

At the same time, the present invention provides a use of mitoxantrone liposome in improving the efficacy of pegaspargase in the treatment of NKTCL.

Further, the present invention provides a drug for treating NKTCL, which is characterized in that it comprises mitoxantrone liposome and pegaspargase.

Preferably, the mitoxantrone liposome of the present invention is preferably mitoxantrone hydrochloride liposome.

The NKTCL in the present invention includes treatment naïve, relapsed, refractory extranodal NKTCL. Preferably, the treatment naïve, relapsed, refractory NKTCL is treatment naïve, relapsed, refractory extranodal nasal NK/T-cell lymphoma.

The drugs may further include other first-line and second-line drugs for treating NKTCL, and the drugs are the first-line and second-line drugs approved by drug regulatory authorities in China or other countries and regions (such as the United States, the European Union, Japan, South Korea, etc.) for treating NKTCL.

Preferably, the drug may be an injection dosage form, including liquid injection, powder for injection, tablet for injection and the like. Mitoxantrone hydrochloride liposome and pegaspargase may be present in the same preparation or in separated preparations. When the mitoxantrone hydrochloride liposome is used as a liquid injection, calculated on the basis of mitoxantrone, it contains 0.5 to 5 mg/ml active ingredient , preferably 1 to 2 mg/ml, more preferably 1 mg/ml. When pegaspargase is an injection, it contains asparaginase in an amount of 1000-5000 IU/5ml, preferably asparaginase 3750 IU/5ml.

Preferably, calculated on the basis of mitoxantrone, the therapeutically effective amount of mitoxantrone hydrochloride liposome is 8 to 30 mg/m², more preferably 12 to 24 mg/m². For example, 12 mg/m², 14 mg/m², 16 mg/m², 18 mg/m², 20 mg/m², 24 mg/m². Preferably, the administration mode of mitoxantrone hydrochloride liposome is administered intravenously.

Preferably, the administration cycle is administered once every 3 weeks. The administration is no more than 6 cycles at most. Preferably, for intravenous administration every time, the infusion time of the liposomal pharmaceutical preparation is 30 min to 120 min, preferably 60 min to 120 min, further preferably 90 ± 15 min.

Calculated on the basis of asparaginase, the dose of pegaspargase is 2000 to 2500 IU/m², preferably in intramuscular injection, and it can be administered at any time before, during and after the administration of mitoxantrone liposome. The administration cycle is the same as that of mitoxantrone hydrochloride liposome.

The present invention further provides a composition for use in treating NKTCL, comprising mitoxantrone hydrochloride liposome and pegaspargase, wherein a therapeutically effective amount of mitoxantrone hydrochloride liposome is administered to NKTCL patients, and 2000~2500 IU/m² of pegaspargase is administered at any time before, during, and after the administration of mitoxantrone liposome.

The present invention further provides a drug for improving the efficacy of pegaspargase in the treatment of NKTCL, wherein the drug comprises mitoxantrone hydrochloride liposome, and the mitoxantrone liposome is administered at any time before, during, and after the administration of pegaspargase, at a dose of 8 to 30 mg/m², administered once every 3 weeks. The dose of mitoxantrone hydrochloride liposome of the present invention is calculated on the basis of mitoxantrone.

The mitoxantrone hydrochloride liposome described in the present invention can be prepared by conventional methods in the art, and the mitoxantrone hydrochloride liposome can be prepared by any method disclosed in the prior art, such as by the method disclosed in WO2008/080367 A1.

In some embodiments, the mitoxantrone hydrochloride liposome of the present invention, with a particle size of about 30 to 80 nm, comprises: 1) the active ingredient mitoxantrone, which can form an insoluble precipitation with multivalent counterion in liposome, 2) lipid bilayer containing phospholipid with a phase transition temperature (Tm) higher than the body temperature, so that the phase transition temperature of the liposome is higher than the body temperature. The phospholipid with the Tm higher than the body temperature is phosphatidylcholine, hydrogenated soy lecithin, hydrogenated ovolecithin, lecithin bis palmitate or lecithin bis stearate or any combination thereof, and the particle size is about 35 to 75 nm, preferably 40 to 70 nm, further preferably 40 to 60 nm, especially preferably 60 nm. The lipid bilayer contains hydrogenated soy lecithin, cholesterol and distearoylphosphatidylethanolamine modified with polyethylene glycol 2000 in a mass ratio of 3:1:1, the particle size is about 60 nm, and the counterion is sulfate ion. Alternatively, the lipid bilayer of the liposome contains hydrogenated soy lecithin, cholesterol and distearoylphosphatidylethanolamine modified with polyethylene glycol 2000 in a mass ratio of 3:1:1, the particle size is about 40 to 60 nm, the counterion is sulfate ion, further, the weight ratio of hydrogenated soy lecithin : cholesterol : distearoylphosphatidylethanolamine modified with polyethylene glycol 2000 : mitoxantrone is 9.58 : 3.19 : 3.19 : 19 : 1.

In some embodiments, the mitoxantrone hydrochloride liposome of the present invention are prepared as follows: HSPC (hydrogenated soy lecithin), Chol (cholesterol) and DSPE-PEG2000 (distearoylphosphatidylethanolamine modified with polyethylene glycol 2000) are weighed in a mass ratio of (3 : 1 : 1), dissolved in 95% ethanol to obtain a clear solution (i.e., ethanol solution of phospholipid). Ethanol solution of phospholipid is mixed with 300 mM ammonium sulfate solution and the resultant is hydrated for 1 hour by shaking at 60 to 65 °C to obtain heterogeneous multilamelar liposome. The microfluidic equipment was then used to reduce the particle size of liposome. After the obtained sample was diluted 200-fold with NaCl solution in a concentration of 0.9%, it was detected with NanoZS, and the average particle size of the particles was about 60 nm, with the main peak concentrated between 40 to 60 nm. Then, the ultrafiltration device was used to remove the ammonium sulfate of the blank liposome external phase, and the external phase was replaced with 290 mM sucrose and 10 mM glycine to form an ammonium sulfate transmembrane gradient. Based on a ratio of liposome to drug of 16:1, mitoxantrone hydrochloride solution (10mg/mL) was added to the blank liposome, and the drug was loaded at 60 to 65 °C. After incubated for approximately l hour, the encapsulation efficiency of approximately 100% can be demonstrated using gel exclusion chromatography. The product obtained from this formulation is named as PLM 60. The weight ratio of HSPC : Chol : DSPE-PEG2000 : mitoxantrone in PLM60 is 9.58 : 3.19 : 3.19 : 1, and the osmotic pressure of sucrose-glycine solution is close to the physiological value.

### Beneficial technical effects

By administering mitoxantrone hydrochloride liposome and pegaspargase in combination to a NKTCL patient, the efficacy of pegaspargase for treating NKTCL in treatment naïve patients and relapsed or refractory patients can be improved, the disease remission rate can be improved, and it has a favorable safety and tolerability, with less toxicity and side effects, especially the complete response rate (CR rate) and partial response rate (PR rate) of the disease are improved, and the disease progression can be controlled.

### Specific embodiment

The following embodiments are specific descriptions of the present invention and should not limit the scope of the present invention.

### Example 1 Clinical study of mitoxantrone hydrochloride liposome injection for treating treatment naïve and relapsed/refractory NKTCL

This was a single-arm, open-label, multicenter phase I/II clinical study in which subjects with treatment naïve or relapsed/refractory extranodal nasal NK/T-cell lymphoma were assigned to receive different doses of mitoxantrone hydrochloride liposome injection and fixed-dose pegaspargase injection, in order to research the safety and tolerability of the combination regimen, determine the optimal dose of mitoxantrone hydrochloride liposomal injection in the combination regimen, and evaluate the efficacy and observe the pharmacokinetic characteristics.

The study was divided into a dose-escalation phase and a dose-expansion phase.

### I. Design of experiments

### 1. Dose-escalation phase

### (1) Study design

The study included a screening period, a treatment period, and a follow-up period. After signing the informed consent form and completing all baseline tests during the screening period, eligible subjects entered the treatment period and were treated by administrating a combination of mitoxantrone hydrochloride liposome injection with pegaspargase injection, and the administration dose of mitoxantrone hydrochloride liposome injection was gradually increased from the low-dose group to the high-dose group, and the administration dose for the subject was the dose of the drug that was being used or would be used at the time of entering this study. The administration regimen was carried out once every 3 weeks, the first cycle of the treatment period was the DLT (dose-limiting toxicity) observation period, and the subject who had completed 3 weeks of treatment and observation could continue the next cycle of administration until progression, death, withdrawal from the study as required by the subject or his/her legal representative, starting on another new treatment , and the end of the entire study (whichever occurred first), and the administration was no more than 6 cycles at most. Efficacy evaluations were performed every 2 cycles (6 weeks) during treatment. All subjects were required to collect PK (pharmacokinetic) blood samples at different time points before and after administration as prescribed by the protocol, and relevant tests were refined during the study to observe safety and tolerability. An end-of-treatment visit was performed 4 weeks after the last administration. After the end of the treatment, the follow-up period would be entered, and progression-free survival (PFS) follow-up was conducted once every 8 weeks in the subjects who had received at least one administration dose, and 5-year overall survival (OS) rates follow-up was conducted every 8 weeks in the subjects who had already progressed and started other new treatment.

### (2) Dose escalation regimen

Mitoxantrone hydrochloride liposome: 12 mg/m² was used as the initial dose (based on mitoxantrone), and 4 dose groups in total of 12 mg/m², 16 mg/m², 20 mg/m², 24 mg/m² were designed.

Subjects with DLT were treated by investigators in accordance with clinical criterion for diagnosis and treatment, and could continue the next cycle of administration by delaying administration for no more than 2 weeks, maintaining the original dose, or reducing one dose level. The administration dose was not allowed to increase for the same subject. Pegaspargase injection: i.e., liposomal asparaginase (PEG-ASP), the administration dose was 2000 IU/m², for intramuscular administration, which could be administered at any time before, during, and after the administration of mitoxantrone hydrochloride liposome.

### (3) Extended administration after completion of the observation period of DLT

Subjects with DLT were treated by investigators in accordance with clinical criterion for diagnosis and treatment, and could continue the next cycle of administration by delaying administration for no more than 2 weeks, maintaining the original dose, or reducing one dose level. The administration dose was not allowed to increase for the same subject.

If MTD (maximum tolerated dose) of mitoxantrone hydrochloride liposome injection was found, MTD was selected as RP2D (phase II recommended dose); If the MTD of mitoxantrone hydrochloride liposome injection was not found, RP2D was determined by the investigator and sponsor in consultation based on the results of the DLT observation period in the dose escalation phase. The RP2D dose was selected for expansion during the dose expansion phase.

### 2. Dose expansion phase

The extended study was conducted in two groups at the RP2D level: the first group included newly treated extranodal nasal type NK/T cell lymphoma, and the second group included relapsed/refractory extranodal NK/T cell lymphoma, and both groups were treated with mitoxantrone hydrochloride liposome injection combined with pegaspargase injection, and in each group 10 to 40 effective cases were expanded to explore the efficacy and tolerability of the combination regimen.

The study included a screening period, a treatment period, and a follow-up period.

After signing the informed consent form and completing all baseline tests during the screening period, eligible subjects entered the treatment phase. The administration regimen was carried out once every 3 weeks, until disease progression, death, withdrawal from the study as required by the subject or his/her legal representative, starting on another new treatment, and the end of the entire study (whichever occurred first), and the administration was no more than 6 cycles at most. Efficacy evaluations were performed every 2 cycles (6 weeks) during treatment. All subjects were required to collect PK (pharmacokinetic) blood samples at different time points before and after administration as prescribed by the protocol, and relevant tests were refined during the study to observe safety and tolerability. An end-of-treatment visit was performed 4 weeks after the last administration. After the end of the treatment, the follow-up period would be entered, and progression-free survival (PFS) follow-up was conducted once every 8 weeks in the subjects who had received at least one administration dose, and 5-year overall survival (OS) rates follow-up was conducted every 8 weeks in the subjects who had already progressed and started other new treatment.

### 3. Study end time

Dose escalation phase: Study ended after RP2D was determined.

Dose expansion phase: The study ended after CR rate (complete response rate), PR rate (partial response rate), ORR (objective response rate), DCR (disease control rate), incidence rate of adverse events during treatment and safety follow-up period, vital signs, physical examination, laboratory test abnormalities, etc. were observed.

### II. Study population

### (1) Inclusion Criteria

Subjects who met all the following inclusion criteria before enrollment:
1. Subject fully understand and voluntarily participate in this study and sign informed consent.
2. Age ≥18, ≤75 years.
3. Histologically confirmed diagnosis of treatment-naive, relapsed or refractory extranodal NK/T-cell lymphoma nasal type (NKTCL)Treatment naïve.
   Relapsed NKTCL was defined by the appearance of any new lesion at the primary site or at other sites after treatment with asparaginase/pegaspargase-containing chemotherapy regimen to achieve complete response (CR).
   Refractory NKTCL was defined as any one of the following: the efficacy evaluation was progressive disease (PD) after two cycles (6 weeks) of treatment with asparaginase/pegaspargase-containing chemotherapy regimen, or the efficacy evaluation failure to achieve partial response (PR) after 4 cycles (12 weeks), or the efficacy evaluation failure to achieve complete response (CR) after 6 cycles (18 weeks).
4. ECOG (Eastern Cooperative Oncology Group) score 0~1;
5. At least one measurable lesion as per Lugano 2014 criteria.
6. The organ function level must meet the following requirements:
   (1) Bone marrow: absolute neutrophil count (ANC) ≥1.5×10⁹/L, hemoglobin ≥80 g/L (no transfusion within 14 days), platelet ≥ 75×10⁹/L;
   (2) liver function: aspartate aminotransferase (AST), alanine aminotransferase (ALT) ≤ 3 times the upper limit of normal (ULN), total bilirubin (TBIL) ≤1.5×ULN (AST, ALT≤5×ULN if documented hepatic involvement of lymphoma);
   (3) Renal function: serum creatinine ≤1.5×ULN;
   (4) Coagulation function: International normalized ratio (INR) and activated partial thromboplastin time (APTT) ≤ 1.5×ULN.

### (II) Exclusion criteria

### Subjects who met any of the following criteria were not eligible for inclusion in this study

1. For NKTCL any of the following situations occurred:
   (1) Known infiltration of the bone marrow according to criteria for leukemia (≥20% myeloblast);
   (2) Lymphoma central invasion;
   (3) Hemophagocyic syndrome.
2. History of allergy and contraindications to mitoxantrone hydrochloride and/or asparaginase/pegaspargase.
3.Chemotherapy, immunotherapy, targeted therapy, endocrine therapy, and radiotherapy within 4 weeks of the first dose of mitoxantrone hydrochloride liposome injection (2 weeks for the local radiation therapy for pain relief).
4.Life expectancy <3 months.
5. For heart function any of the following conditions occurred:
   (1) Impaired cardiac function or serious heart disease: myocardial infarction, congestive heart failure, heart disease with symptoms requiring drug treatment such as unstable angina and arrhythmia, uncontrolled severe hypertension within 6 months before screening, viral myocarditis or history of persistent cardiomyopathy within 6 months;
   (2) Cardiac function grades III ~ IV;
   (3) Left ventricular ejection fraction < 50% on echocardiography;
   (4) In the absence of a pacemaker, degree II/III of atrioventricular block, long QT syndrome, or QTc >480 ms was present on 12-lead ECG.
6. Infectious disease screening: hepatitis B surface antigen was positive and HBV-DNA titer was above the upper limit of the study center, hepatitis C virus antibody was positive and HCV-RNA titer was above the upper limit of the study center, human immunodeficiency virus (HIV) antibody was positive in initial screening.
7.Acute symptomatic or chronic pancreatitis within 4 weeks prior to screening.
8.History of solid organ transplantation, autologous hematopoietic stem cell transplantation within 6 months prior to screening, or allogeneic hematopoietic stem cell transplantation before screening.
9. History of, or known additional tumor (exception: non-melanoma skin cancer (in situ) and cervical cancer (in situ) which had been cured and have not recurred within 5 years).
10. Major surgery within 4 weeks prior to screening or had a surgical schedule during the study period.
11.A serious infection within 4 weeks prior to screening and not suitable for the study according to the judgment of the investigator.
12. Uncontrolled diabetes at screening.
13. History of active bleeding within 3 months prior to screening.
14. Known drug abuse (use of narcotic drugs or psychotropic drugs for non-medical purposes) or drug dependence (sedative-hypnotics, analgesics, narcotics, stimulants, psychotomimetic drugs, etc.).
15. Known psychiatric disorders or cognition disorders.
16. Pregnant or breastfeeding women, or patients who were expecting to conceive or father in 12 months
17. Not suitable for this study as determined by the investigator due to other reasons.

### (III) Discontinuation /Termination Criteria

Subjects who met any of the follow criteria during the study would be withdrawn from the study.
1. Subject declined further study participation.
2. Subject was non-compliant with protocol based on investigator and/or sponsor's assessment.
3. Any adverse events which, in the opinion or the investigator, indicated that continued participation in the study would increase the risk for the subject.
4. Subjects had disease progression after administration.
5. Pregnancy.

### III. Results of the study

Disease assessment was performed utilizing PET-CT, tumor responses were assessed according to Lugano 2014 criteria.

A total of 24 subjects were enrolled in this study, including 15 subjects with treatment naïvetreatment-naive extranodal NK/T-cell lymphoma (nasal type) and 9 subjects with relapsed/refractory extranodal NK/T-cell lymphoma (nasal type), of which subjects who underwent one efficacy evaluation (6 weeks) were enrolled for the following assessment:
Group I: 11 subjects withtreatment naïve treatment-naive extranodal NK/T-cell lymphoma (nasal type).
Group II: 9 subjects with relapsed/refractory extranodal NK/T-cell lymphoma(nasal type). The dosage of mitoxantrone hydrochloride liposome was shown in Table 1 below, and the dosage of pegaspargase was 2000 IU/m².

The results of the evaluation were shown in Table 1 below:

**Table 1**

| | Treatment-naive treatment naïve subjects | | | | | Relapsed/refractory subjects | | | | |
|---|---|---|---|---|---|---|---|---|---|---|
| Dosage | 12mg/m² | 16mg/m² | 20mg/m² | 24mg/m² | Total | 12mg/m² | 16mg/m² | 20mg/m² | 24mg/m² | Total |
| CR (complete response) | 2 | 2 | 4 | 1 | 9 | 0 | 1 | 2 | 0 | 3 |
| PR (partial response) | 0 | 0 | 0 | 2 | 2 | 0 | 2 | 0 | 2 | 4 |
| SD (Stable Disease) | 0 | 0 | 0 | 0 | 0 | 1 | 1 | 0 | 0 | 2 |
| PD (Progressive Disease) | 0 | 0 | 0 | 0 | 0 | 0 | 0 | 0 | 0 | 0 |
| CR rate | 81.8% (9/11) | | | | | 33.3% (3/9) | | | | |
| PR rate | 18.2% (2/11) | | | | | 44.4% (4/9) | | | | |
| ORR (Objective Response Rate) | 100% (11/11) | | | | | 77.8% (7/9) | | | | |
| DCR (Disease Control Rate) | 100% (11/11) | | | | | 100% (9/9) | | | | |

| | | | | | | | | | | |
|---|---|---|---|---|---|---|---|---|---|---|
| Results: The treatment of mitoxantrone hydrochloride liposome combined with pegaspargase was safe and well tolerated in both treatment-naive subjects and relapse/refractory subject group, with less toxicities and significant curative effects, in particular, the CR rate and PR rate of different subjects can be improved, and the disease control rate was 100%, and there was no case of disease progression during treatment. It indicated that the administration of mitoxantrone hydrochloride liposome combined with pegaspargase had broad clinical application prospects in the treatment of NKTCL. | | | | | | | | | | |

The foregoing is only a specific embodiment of the present invention, but the scope of protection of the present invention is not limited thereto, and any change or replacement which is easily conceived of by a person skilled in the art who is familiar with the art should belong to the scope of the technology disclosed in the present invention. The scope of protection is as outlined in the claims.

### Practicability

The pharmaceutical composition provided according to the present invention may be used for the treatment of NKTCL.

## Claims

1. Mitoxantrone liposome and pegaspargase for use in treating NK/T-cell lymphoma.

2. Mitoxantrone liposome and pegaspargase for use according to claim 1, wherein the efficacy of pegaspargase in treating NK/T-cell lymphoma is improved.

3. Mitoxantrone liposome and pegaspargase for use according to claim 1 or 2, wherein the NK/T-cell lymphoma includes treatment of naive, relapsed, refractory extranodal NK/T-cell lymphoma; preferably, the treatment of naive, relapsed, refractory extranodal NK/T-cell lymphoma is a treatment of naive, relapsed, refractory extranodal nasal NK/T-cell lymphoma.

4. Mitoxantrone liposome and pegaspargase for use according to any one of claims 1 to 3, wherein mitoxantrone liposome and pegaspargase are in an injection dosage form, including liquid injection, powder for injection, tablet for injection.

5. Mitoxantrone liposome and pegaspargase for use according to any one of claims 1 to 4, wherein mitoxantrone liposome and pegaspargase are present in the same preparation, or in separated preparations.

6. Mitoxantrone liposome and pegaspargase for use according to any one of claims 1 to 5, wherein the mitoxantrone liposome is liquid injection, calculated on the basis of mitoxantrone, it contains 0.5 to 5 mg/ml active ingredient, preferably 1 to 2 mg/ml, more preferably 1 mg/ml.

7. Mitoxantrone liposome and pegaspargase for use according to any one of claims 1 to 6, wherein pegaspargase is a liquid injection containing asparaginase in an amount of 1000 to 5000 IU/5 ml, preferably asparaginase 3750 IU/5 ml.

8. Mitoxantrone liposome and pegaspargase for use according to any one of claims 1 to 7, wherein the drug further comprises other drug for treating NK/T-cell lymphoma.

9. Mitoxantrone liposome and pegaspargase for use according to any one of claims 1 to 8, wherein therapeutically effective amounts of mitoxantrone liposome and pegaspargase are administered to a patient with NK/T-cell lymphoma, and the administration is preferably injection form; preferably, calculated on the basis of mitoxantrone, the therapeutically effective amount of mitoxantrone hydrochloride liposome is 8 to 30 mg/m², and the administration cycle is once every 3 weeks; the dosage of pegaspargase is 2000 to 2500 IU/m², preferably administered intramuscularly.

10. Mitoxantrone liposome and pegaspargase for use according to any one of claims 1 to 9, wherein the mitoxantrone liposome is mitoxantrone hydrochloride liposome.

11. Mitoxantrone liposome and pegaspargase for use according to any one of claims 1 to 10, wherein the particle size of mitoxantrone hydrochloride liposome is about 30 to 80 nm, preferably about 35 to 75 nm, more preferably about 40 to 70 nm, further preferably about 40 to 60 nm, and mitoxantrone hydrochloride liposome contains: 1) the active ingredient mitoxantrone, 2) lipid bilayer containing phospholipid with a phase transition temperature (Tm) higher than body temperature, wherein the phospholipid with the Tm higher than the body temperature is phosphatidylcholine, hydrogenated soy lecithin, hydrogenated ovolecithin, lecithin bis palmitate, lecithin bis stearate and any combination thereof.

12. A composition for use in treating NK/T-cell lymphoma, comprising mitoxantrone hydrochloride liposome and pegaspargase, wherein mitoxantrone hydrochloride liposome is administered in an amount of 8 to 30 mg/m² to a patient with NK/T-cell lymphoma, and the administration cycle is once every 3 weeks; and pegaspargase is administered in an amount of 2000 to 2500 IU/m² at any time before, during and after the administration of mitoxantrone liposome.

13. The composition for use according to claim 12, wherein the composition contains mitoxantrone hydrochloride liposome and pegaspargase, and wherein the mitoxantrone liposome is administered at any time before, during and after the administration of pegaspargase, at a dosage of 8-30 mg/m², administered once every 3 weeks.

14. The composition for use according to claim 12 or 13, wherein the particle size of mitoxantrone hydrochloride liposome is about 30 to 80 nm, preferably about 35 to 75 nm, more preferably about 40 to 70 nm, further preferably about 40 to 60 nm, and mitoxantrone hydrochloride liposome contains: 1) the active ingredient mitoxantrone, 2) lipid bilayer containing phospholipid with a phase transition temperature (Tm) higher than body temperature, and the phospholipid with the Tm higher than body temperature is phosphatidylcholine, hydrogenated soy lecithin, hydrogenated ovolecithin, lecithin bis palmitate, lecithin bis stearate and any combination thereof.

## Patentansprüche

1. Mitoxantron-Liposom und Pegaspargase zur Verwendung bei der Behandlung von NK/T-Zell-Lymphomen.

2. Mitoxantron-Liposom und Pegaspargase zur Verwendung nach Anspruch 1, wobei die Wirksamkeit der Pegaspargase bei der Behandlung von NK/T-Zelle-Lymphomen verbessert wird.

3. Mitoxantron-Liposom und Pegaspargase zur Verwendung nach Anspruch 1 oder 2, wobei das NK/T-Zell-Lymphom die Behandlung eines naiven, rezidivierten, refraktären extranodalen NK/T-Zell-Lymphoms beinhaltet; wobei vorzugsweise die Behandlung eines naiven, rezidivierten, refraktären extranodalen NK/T-Zell-Lymphoms eine Behandlung eines naiven, rezidivierten, refraktären extranodalen nasalen NK/T-Zell-Lymphoms ist.

4. Mitoxantron-Liposom und Pegaspargase zur Verwendung nach einem der Ansprüche 1 bis 3, wobei Mitoxantron-Liposom und Pegaspargase in einer Injektions-Dosierungsform vorliegen, was eine flüssige Injektion, ein Pulver zur Injektion und eine Tablette zur Injektion beinhaltet.

5. Mitoxantron-Liposom und Pegaspargase zur Verwendung nach einem der Ansprüche 1 bis 4, wobei Mitoxantron-Liposom und Pegaspargase in derselben Zubereitung oder in getrennten Zubereitungen vorliegen.

6. Mitoxantron-Liposom und Pegaspargase zur Verwendung nach einem der Ansprüche 1 bis 5, wobei das Mitoxantron-Liposom eine flüssige Injektion ist, die auf Basis von Mitoxantron berechnet 0,5 bis 5 mg/ml, vorzugsweise 1 bis 2 mg/ml, noch bevorzugter 1 mg/ml aktiven Wirkstoff enthält.

7. Mitoxantron-Liposom und Pegaspargase zur Verwendung nach einem der Ansprüche 1 bis 6, wobei Pegaspargase in einer flüssigen Injektion vorliegt, die Asparaginase in einer Menge von 1.000 bis 5.000 IU/5 ml, vorzugsweise 3.750 IU/5 ml Asparaginase enthält.

8. Mitoxantron-Liposom und Pegaspargase zur Verwendung nach einem der Ansprüche 1 bis 7, wobei das Arzneimittel ferner andere Arzneimittel zur Behandlung von NK/T-Zell-Lymphomen enthält.

9. Mitoxantron-Liposom und Pegaspargase zur Verwendung nach einem der Ansprüche 1 bis 8, wobei therapeutisch wirksame Mengen an Mitoxantron-Liposom und Pegaspargase an einen Patienten mit NK/T-Zell-Lymphom verabreicht werden, und die Verabreichung vorzugsweise in Form einer Injektion erfolgt; wobei die therapeutisch wirksame Menge an Mitoxantron-Hydrochlorid-Liposom auf Grundlage von Mitoxantron vorzugsweise 8 bis 30 mg/ml beträgt und der Verabreichungszyklus einmal alle 3 Wochen ist; die Dosierung von Pegaspargase 2.000 bis 2.500 IU/m2 beträgt, und vorzugsweise intramuskulär verabreicht wird.

10. Mitoxantron-Liposom und Pegaspargase zur Verwendung nach einem der Ansprüche 1 bis 9, wobei das Mitoxantron-Liposom Mitoxantron-Hydrochlorid-Liposom ist.

11. Mitoxantron-Liposom und Pegaspargase zur Verwendung nach einem der Ansprüche 1 bis 10, wobei die Partikelgröße des Mitoxantron-Hydrochlorid-Liposoms etwa 30 bis 80 nm, vorzugsweise etwa 35 bis 75 nm, noch bevorzugter etwa 40 bis 70 nm, sogar noch bevorzugter etwa 40 bis 60 nm beträgt, und das Mitoxantron-Hydrochlorid-Liposom enthält: 1) den aktiven Wirkstoff Mitoxantron, 2) eine Lipid-Doppelschicht enthaltend Phospholipid mit einer Phasenübergangstemperatur (Tm), die höher ist als Körpertemperatur, wobei das Phospholipid mit einer Phasenübergangstemperatur (Tm), die höher ist als Körpertemperatur, Phosphatidylcholin, hydriertes Sojalecithin, hydriertes Ovolecithin, Lecithin-bis-Palmitat, Lecithin-bis-Stearat und eine beliebige Kombination davon ist.

12. Zusammensetzung zur Verwendung bei der Behandlung von NK/T-Zell-Lymphomen, umfassend Mitoxantron-Hydrochlorid-Liposom und Pegaspargase, wobei das Mitoxantron-Hydrochlorid-Liposom in einer Menge von 8 bis 30 mg/ml an einen Patienten mit NK/T-Zell-Lymphom verabreicht wird und der Verabreichungszyklus einmal alle 3 Wochen ist; und Pegaspargase in einer Menge von 2.000 bis 2.500 IU/m2 zu einem beliebigen Zeitpunkt vor, während und nach der Verabreichung von Mitoxantron-Liposom verabreicht wird.

13. Zusammensetzung zur Verwendung nach Anspruch 12, wobei die Zusammensetzung Mitoxantron-Hydrochlorid-Liposom und Pegaspargase enthält, und wobei Mitoxantron-Liposom in einer Dosierung von 8-30 mg/ml zu einem beliebigen Zeitpunkt vor, während und nach der Verabreichung von Pegaspargase verabreicht wird, und einmal alle 3 Wochen verabreicht wird.

14. Zusammensetzung zur Verwendung nach Anspruch 12 oder 13, wobei die Partikelgröße des Mitoxantron-Hydrochlorid-Liposoms etwa 30 bis 80 nm, vorzugsweise etwa 35 bis 75 nm, noch bevorzugter etwa 40 bis 70 nm, sogar noch bevorzugter etwa 40 bis 60 nm beträgt, und das Mitoxantron-Hydrochlorid-Liposom enthält: 1) den aktiven Wirkstoff Mitoxantron, 2) eine Lipid-Doppelschicht enthaltend Phospholipid mit einer Phasenübergangstemperatur (Tm), die höher ist als Körpertemperatur, und das Phospholipid mit einer Phasenübergangstemperatur (Tm), die höher ist als Körpertemperatur, Phosphatidylcholin, hydriertes Sojalecithin, hydriertes Ovolecithin, Lecithin-bis-Palmitat, Lecithin-bis-Stearat und eine beliebige Kombination davon ist.

## Revendications

1. Liposome de mitoxantrone et pégaspargase destinés à être utilisés dans le traitement d'un lymphome à cellules NK/T.

2. Liposome de mitoxantrone et pégaspargase destinés à être utilisés selon la revendication 1, dans lesquels l'efficacité de la pégaspargase dans le traitement d'un lymphome à cellules NK/T est amélioré.

3. Liposome de mitoxantrone et pégaspargase destinés à être utilisés selon la revendication 1 ou 2, dans lesquels le lymphome à cellules NK/T comprend le traitement d'un lymphome à cellules NK/T extranodal naïf, récurrent et réfractaire, de préférence le traitement d'un lymphome à cellules NK/T extranodal naïf, récurrent et réfractaire est un traitement d'un lymphome à cellules NK/T nasal extranodal naïf, récurrent et réfractaire.

4. Liposome de mitoxantrone et pégaspargase destinés à être utilisés selon l'une quelconque des revendications 1 à 3, dans lesquels le liposome de mitoxantrone et la pégaspargase se présentent sous une forme de dosage d'injection, y compris une injection liquide, une poudre pour injection, un comprimé pour injection.

5. Liposome de mitoxantrone et pégaspargase destinés à être utilisés selon l'une quelconque des revendications 1 à 4, dans lesquels le liposome de mitoxantrone et la pégaspargase sont présents dans la même préparation, ou dans des préparations distinctes.

6. Liposome de mitoxantrone et pégaspargase destinés à être utilisés selon l'une quelconque des revendications 1 à 5, dans lesquels le liposome de mitoxantrone est une injection liquide, calculée par rapport à la mitoxantrone, elle contient 0,5 à 5 mg/ml de principe actif, de préférence 1 à 2 mg/ml, plus préférablement 1 mg/ml.

7. Liposome de mitoxantrone et pégaspargase destinés à être utilisés selon l'une quelconque des revendications 1 à 6, dans lesquels la pégaspargase est une injection liquide contenant de l'asparaginase en une quantité de 1000 à 5000 UI/5 ml, de préférence de l'asparaginase à 3750 UI/5 ml.

8. Liposome de mitoxantrone et pégaspargase destinés à être utilisés selon l'une quelconque des revendications 1 à 7, dans lesquels le médicament comprend en outre un autre médicament destiné au traitement d'un lymphome à cellules NK/T.

9. Liposome de mitoxantrone et pégaspargase destinés à être utilisés selon l'une quelconque des revendications 1 à 8, dans lesquels les quantités thérapeutiquement efficaces de liposome de mitoxantrone et de pégaspargase sont administrées à un patient atteint d'un lymphome à cellules NK/T, et l'administration est de préférence une forme d'injection ; de préférence, calculée par rapport à la mitoxantrone, la quantité thérapeutiquement efficace de liposome de chlorhydrate de mitoxantrone est de 8 à 30 mg/m², et le cycle d'administration est une fois toutes les 3 semaines ; le dosage de la pégaspargase est de 2000 à 2500 UI/m², de préférence administré par voie intramusculaire.

10. Liposome de mitoxantrone et pégaspargase destinés à être utilisés selon l'une quelconque des revendications 1 à 9, dans lesquels le liposome de mitoxantrone est le liposome de chlorhydrate de mitoxantrone.

11. Liposome de mitoxantrone et pégaspargase destinés à être utilisés selon l'une quelconque des revendications 1 à 10, dans lesquels la taille de particule du liposome de chlorhydrate de mitoxantrone est d'environ 30 à 80 nm, de préférence d'environ 35 à 75 nm, plus préférablement d'environ 40 à 70 nm, en outre de préférence d'environ 40 à 60 nm, et le liposome de chlorhydrate de mitoxantrone contient : 1) le principe actif mitoxantrone, 2) une bicouche lipidique contenant un phospholipide avec une température de transition de phase (Tm) supérieure à la température corporelle, dans lequel le phospholipide avec la Tm supérieure à la température corporelle est la phosphatidylcholine, la lécithine de soja hydrogénée, l'ovolécithine hydrogénée, le bis palmitate de lécithine, le bis stéarate de lécithine et toute combinaison de ceux-ci.

12. Composition destinée à être utilisée dans le traitement d'un lymphome à cellules NK/T, comprenant du liposome de chlorhydrate de mitoxantrone et de la pégaspargase, dans laquelle le liposome de chlorhydrate de mitoxantrone est administré en une quantité de 8 à 30 mg/m² à un patient atteint d'un lymphome à cellules NK/T, et le cycle d'administration est une fois toutes les 3 semaines ; et la pégaspargase est administrée en une quantité de 2000 à 2500 UI/m² à tout moment avant, pendant et après l'administration du liposome de mitoxantrone.

13. Composition destinée à être utilisée selon la revendication 12, dans laquelle la composition contient du liposome de chlorhydrate de mitoxantrone et de la pégaspargase, et dans laquelle le liposome de chlorhydrate de mitoxantrone est administré à tout moment avant, pendant et après l'administration de la pégaspargase, à un dosage de 8 à 30 mg/m², administré une fois toutes les 3 semaines.

14. Composition destinée à être utilisée selon la revendication 12 ou 13, dans laquelle la taille de particule du liposome de chlorhydrate de mitoxantrone est d'environ 30 à 80 nm, de préférence d'environ 35 à 75 nm, plus préférablement d'environ 40 à 70 nm, en outre de préférence d'environ 40 à 60 nm, et le liposome de chlorhydrate de mitoxantrone contient : 1) le principe actif mitoxantrone, 2) une bicouche lipidique contenant un phospholipide avec une température de transition de phase (Tm) supérieure à la température corporelle, et le phospholipide avec la Tm supérieure à la température corporelle est la phosphatidylcholine, la lécithine de soja hydrogénée, l'ovolécithine hydrogénée, le bis palmitate de lécithine, le bis stéarate de lécithine et toute combinaison de ceux-ci.
